Europäisches Patentamt

European Patent Office

Office européen des brevets

(11) Veröffentlichungsnummer: **0 366 827**

**A1**

(12) **EUROPÄISCHE PATENTANMELDUNG**

(21) Anmeldenummer: **88118397.4**

(22) Anmeldetag: **04.11.88**

(51) Int. Cl.5: **A61L 2/00 , A61L 2/10**

(43) Veröffentlichungstag der Anmeldung:
**09.05.90 Patentblatt 90/19**

(84) Benannte Vertragsstaaten:
**AT CH DE FR IT LI**

(71) Anmelder: **THÖBEN, Helga**
**Beethovenstrasse 17**
**D-4594 Garrel(DE)**

(72) Erfinder: **THÖBEN, Helga**
**Beethovenstrasse 17**
**D-4594 Garrel(DE)**

(74) Vertreter: **Hoormann, Walter, Dr.-Ing. et al**
**FORRESTER & BOEHMERT**
**Widenmayerstrasse 4/I**
**D-8000 München 22(DE)**

(54) **Desinfiziervorrichtung insbesondere für Krankenhaus-Betten.**

(57) Die Erfindung betrifft eine Vorrichtung (1) zum Deinfizieren von insbesondere in Krankenhäusern od.dgl. befindlichen Gegenständen wie Rollstühlen, Strecktischen, Rehabilitationsgeräten, Instrumentenwagen etc., insbesondere Betten (2), mit einem verschließbaren Gehäuse (3), in dem wenigstens eine Desinfiziereinrichtung (17, 26) angeordnet ist, wobei das auf Rollen (8, 9) od.dgl. verfahrbare Gehäuse (3) aus wenigstens zwei Längsabschnitten (4, 6) besteht, die aus ihrer Betriebsstellung in Längsrichtung (7) der Vorrichtung (1) unter Verkürzung der Betriebslänge der Vorrichtung (1) relativ zueinander beweglich sind.

FIG.5

EP 0 366 827 A1

## Desinfiziervorrichtung, insbesondere für Krankenhaus-Betten

Die Erfindung betrifft eine Vorrichtung zum Desinfizieren von insbesondere in Krankenhäusern od.dgl. befindlichen Gegenständen wie Rollstühlen, Strecktischen, Rehabilitationsgeräten, Instrumentenwagen etc., insbesondere Betten, mit einem verschließbaren Gehäuse zur Aufnahme wenigstens eines zu desinfizierenden Gegenstandes, wobei in dem Gehäuse wenigstens eine Desinfiziereinrichtung angeordnet ist.

Eine Entkeimung von Gegenständen wie Betten etc. durch Desinfizierung ist aus hygienischen Gründen an sich in zahlreichen Fällen (bspw. in Hotels, Jugendherbergen etc.) geboten, spielt jedoch insbesondere in Krankenhäusern, Rehabilitationszentren etc. eine besondere Rolle, da dort einerseits eine besonders hohe Konzentration schädlicher Bakterien gegeben ist, und da es sich bei den Patienten derartiger Anstalten in aller Regel um Menschen handelt, deren Infektionsanfälligkeit aufgrund einer krankheitsbedingten Schwächung ihres Immunsystems besonders hoch ist.

Dieses Erfordernis gilt praktisch für sämtliche in Krankenhäusern u.dgl. in Gebrauch befindliche Gegenstände wie Rollstühle, Strecktische, Rehabilitationsgeräte, Instrumentenwagen etc., insbesondere jedoch für die Betten, da Krankenhauspatienten bekanntlich regelmäßig die meiste Zeit im Bett verbringen, und da sich aufgrund der Körperwärme, durch Transpiration verdunstete Feuchtigkeit etc. regelmäßig geradezu Idealbedingungen für eine Vermehrung von Bakterien einstellen.

In ordentlich geführten Krankenhäusern u.dgl. werden daher die Patientenbetten nach einem Benutzerwechsel üblicherweise in einen Reinigungs- bzw. Desinfizierraum verbracht und dort sorgfältig gereinigt und desinfiziert. Dieses ist jedoch insbesondere aufgrund der (insbesondere in großen Krankenhäusern od.dlg.) damit verbundenen langen Transportwege ein mühseliges und zeitraubendes Unterfangen, welches darüber hinaus zur Folge hat, daß die theoretisch möglichen Belegungszeiten von Krankenhausbetten insbesondere in Stationen mit relativ hoher Wechselfrequenz nicht annähernd auszunutzen sind.

In nicht optimal geführten Krankenhäusern u.dgl. kommt es daher auch durchaus vor, daß ein Krankenbett bei einem Patientenwechsel nach lediglich konventioneller Reinigung im Krankenzimmer wieder in Benutzung genommen wird, ohne daß es ordnungsgemäß desinfiziert wird. Dieses kann aber - wie ausgeführt - für den Nachfolgepatienten geradezu verheerende Folgen haben, und zwar insbesondere dann, wenn es sich dabei um Patienten mit offenen Wunden bzw. frisch operierte Patienten handelt.

Entsprechendes gilt nicht nur - wie ebenfalls bereits oben angedeutet worden ist - für andere in Krankenhäusern in Gebrauch befindliche Geräte, sondern auch für Beherbergungsstätten wie Hotels, Jugendherbergen etc. zumindest bzgl. der dort in Benutzung befindlichen Betten.

Der vorliegenden Erfindung liegt die Aufgabe zugrunde, eine mobile Disinfiziervorrichtung zu schaffen, mittels welcher Gegestände wie insbesondere Betten an ihrem vorgesehenen Standort mühelos und vor allem schnell gründlich zu reinigen und optimal zu desinfizieren sind.

Die Lösung dieser Aufgabe erfolgt erfindungsgemäß dadurch, daß das auf Rollen od.dgl. verfahrbare Gehäuse aus wenigstens zwei Gehäuse-Längsabschnitten besteht, die aus ihrer ausgefahreren Betriebsstellung in Längsrichtung der Vorrichtung, unter Verkürzung der Betriebslänge der Vorrichtung relativ zueinander in eine Transport- bzw. Ruhestellung beweglich sind, so daß sich dadurch eine mobile Desinfiziervorrichtung ergibt, die in Transportstellung mühelos beweglich und aufgrund ihrer geringen Dimensionen ohne weiteres und leicht von einer einzigen von einem Einsatzort zum anderen zu verbringen, die es aber andererseits gestattet, auch erheblich größere Gegenstände wie bspw. Betten ordnungsgemäß und optimal zu desinfizieren.

Bevorzugt sind die Gehäuse-Längsabschnitte relativ zueinander teleskopierbar ausgebildet, wie weiter unten noch im einzelnen erläutert wird, obwohl zur Lösung der obigen Aufgabe auch andere Möglichkeiten bestehen, nämlich bspw. eine Ausbildung wenigstens eines Gehäuse-Längsabschnittes als Faltenbalg od.dgl.

Um die obige Aufgabe und damit die an eine derartige mobile Desinfiziervorrichtung zu stellenden Anforderungen optimal erfüllen zu können, besteht das Gehäuse bevorzugt aus mehr als zwei Gehäuse-Längsabschnitten, und zwar bevorzugt aus einem Gehäuse-Kopfabschnitt sowie mehreren Gehäuse-Segmenten. Dabei kann der im wesentlichen stets unverändert verbleibende Gehäuse-Kopfabschnitt diverse Gegenstände und Einrichtungen aufnehmen, die für eine optimale, mobile Desinfiziervorrichtung unverzichtbar oder zumindest höchst wünschenswert sind (also z.B. eine ggf. einen Rechner aufweisende Steuereinrichtung, mittels welcher der Ablauf eines Desinfektionsprozesses automatisch zu steuern ist, Hilfsgeräte und Einrichtungen, wie sie nachstehend noch im einzelnen beschrieben werden, sowie Fächer, Schubladen od.dgl. zur Aufnahme weiterer Gegenstände wie Arbeitshandschuhe, Tücher etc.), während die Gehäuse-Segmente gerätemäßig weniger belastet sind, um ihr relatives Zusammenfahren in eine Transport- bzw. Ruhestellung ermöglichen zu können, wie dieses weiter unten ebenfalls noch im einzelnen erläutert wird.

Weitere bevorzugte Ausgestaltungen der vorliegenden Erfindung sind in den Unteransprüchen beschrieben.

Die Erfindung wird nachstehend - auch bzgl. ganz allgemeiner, nicht allein auf die Ausführungsbeispiele bezogene Aspekte - nachstehend an Ausführungsbeispielen unter Bezugnahme auf eine Zeichnung weiter erläutert. Es zeigt:

Fig. 1 eine perspektivische Darstellung einer erfindungsgemäßen Desinfiziervorrichtung in Transport- bzw. Ruhestellung schräg von vorn rechts gesehen;

Fig. 1a den in Fig. 1 mit einer strichpunktierten Linie eingerahmten Absch. la bei Betriebsaufnahme;

Fig. 2 die Vorrichtung gemäß den Fig. 1 und 1a in Betriebsstellung;

Fig. 3 die Vorrichtung gemäß den Fig. 1 und 2 (noch) in Transportstellung mit betriebsfertigem Staubsauger;

Fig. 4 eine perspektivische Darstellung der in Transport- bzw. Ruhestellung befindlichen Vorrichtung gemäß den Fig. 1 bis 3 schräg von links hinten gesehen;

Fig. 5 eine Fig. 2 entsprechende Darstellung der Vorrichtung gemäß den Fig. 1 bis 4 in Betriebsstellung, jedoch schräg von hinten links gesehen mit noch geöffneter Jalousie am rückwärtigen Ende der Vorrichtung;

Fig. 5a eine gegenüber Fig. 5 etwas verkleinerte Darstellung mit geschlossener Jalousie;

Fig. 6 eine Fig. 3 entsprechende Darstellung, jedoch schräg von vorn links gesehen mit geöffneter Seitentür;

Fig. 7 eine rückwärtige Ansicht auf die Darstellung gemäß Fig. 1 in Richtung des Pfeiles VII in Fig. 1 gesehen;

Fig. 8 eine Explosionsdarstellung einer (in Fig. 4 erkennbaren) Filtereinrichtung;

Fig. 9 eine Schnittdarstellung einer Mehrfachsprühdüse zum Versprühen von flüssigen Desinfektionsmitteln;

Fig. 10 mehrere UV-Strahler der Vorrichtung gemäß den Fig. 1 bis 9; und

Fig. 11 eine schematisierte Darstellung der Vorrichtung gemäß den Fig. 1 bis 10 mit einem in der Vorrichtung befindlichen, zu desinfizierenden Bett.

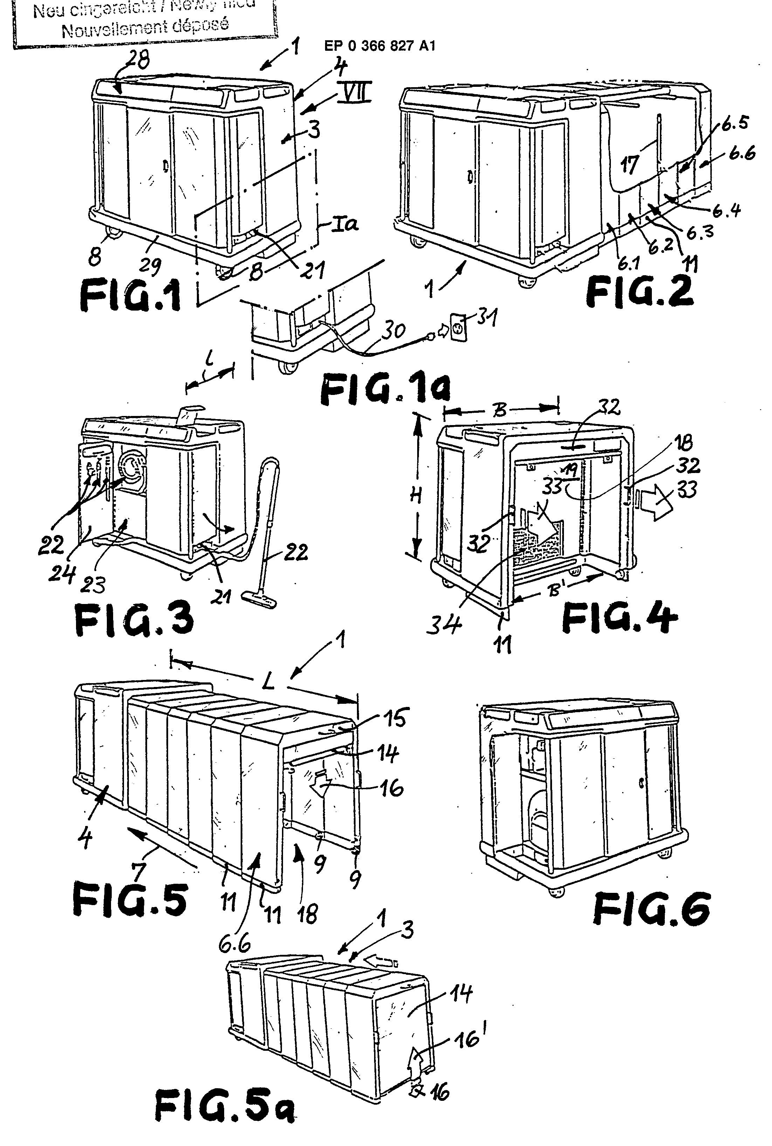

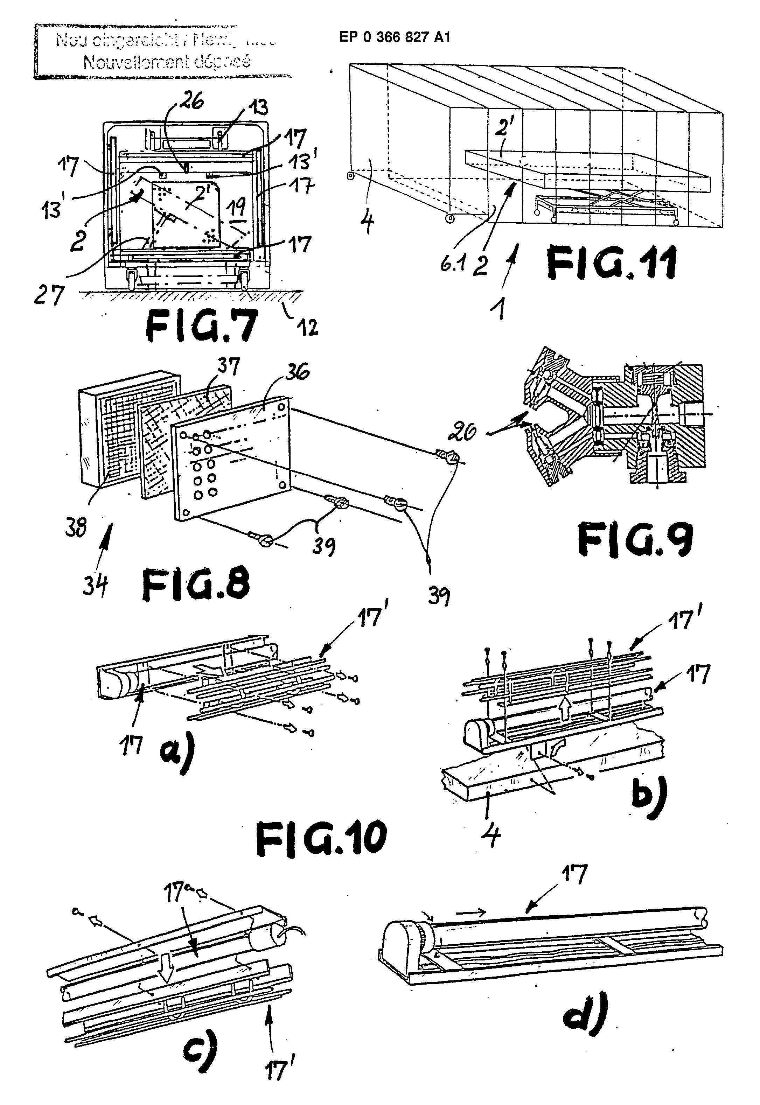

Fig. 1 zeigt eine im ganzen mit 1 bezeichnete Vorrichtung zum Desinfizieren von in Krankenhäusern od.dgl. befindlichen Gegenständen wie Rollstühlen, Strecktischen, Rehabilitationsgeräten, Instrumentenwagen etc., insbesondere jedoch von Betten 2 (s. Fig. 7, 11). Die Vorrichtung 1 weist ein verschließbares Gehäuse 3 zur Aufnahme wenigstens eines zu desinfizierenden Gegenstandes 2 auf, in dem wenigstens eine Desinfiziereinrichtung angeordnet ist, wie dieses weiter unten noch im einzelnen beschrieben wird.

Das Gehäuse 3 besteht insgesamt aus sieben Längsabschnitten, und zwar aus einem Gehäusekopfabschnitt 4 sowie sechs Gehäuse-Segmenten 6.1 bis 6.6 (s. Fig. 2), die aus ihrer Transport- bzw. Ruhestellung gemäß Fig. 1 in Längsrichtung 7 (s. Fig. 5) der Vorrichtung 1 unter Vergrößerung der Betriebslänge 1 der Vorrichtung 1 relativ zueinander in eine Betriebsstellung (s. z.B. Fig. 2) beweglich sind. Dabei beträgt die Länge 1 der Vorrichtung 1 in der Transport- bzw. Ruhestellung ca. 70 cm, während die Betriebslänge L ca. 2,8 m beträgt.

An dieser Stelle sei sogleich darauf verwiesen, daß die Breite B der Vorrichtung ebenso wie deren Höhe H (s. Fig. 4) jeweils ca. 1,4 m beträgt.

Die Verlängerungs- bzw. Verkürzungsmöglichkeit der Vorrichtung 1 wird dadurch erreicht, daß die Gehäuselängsabschnitte 4, 6 teleskopierbar ausgebildet sind, und zwar derart, daß in der bspw. in Fig. 1 dargestellten Transport- bzw. Ruhestellung sämtliche Gehäuse-Segmente 6.1 bis 6.6 innerhalb des Gehäuse-Kopfabschnittes 4 unterzubringen sind, wie weiter unten noch weiter erläutert wird.

Der Gehäuse-Kopfabschnitt 4 ist auf Laufrollen 8 verfahrbar abgestützt, während die Gehäuse-Segmente 6 jeweils auf kleineren Laufröllchen 9 (s. Fig. 5) verfahrbar abgestützt sind, die von außen allerdings nicht bzw. kaum erkennbar sind, da jeweils an den unten befindlichen freien Randabschnitten der Gehäuse-Segmente 6 (übrigens ebenso wie den ensprechenden Randabschnitten des Gehäuse-Kopfabschnittes 4) schürzenförmige Dichtelemente 11 angeordnet sind, die sich in der Betriebsstellung der Vorrichtung 1 (s. z.B. Fig. 1) im wesentlichen bis zum Untergrund 12 erstrecken und vorzugsweise in der Betriebsstellung dichtend auf dem Untergrund 12 aufliegen. Dieses ist höchst zweckmäßig, um den Innenraum der Vorrichtung 1 während des Betriebes praktisch gegen die Umgebung abzudichten, und bei der dargestellten Ausführungsform demgemäß sogar erforderlich, weil die Gehäuse-Segmente 6 umgekehrt U-förmig ausgebildet sind (um eine besonders zweckmäßige Teleskopierbarkeit verwirklichen zu können).

Bezgl. der Dichtelemente 11 sei noch nachgetragen, daß diese in Längsrichtung 7 der Vorrichtung 1 unbeweglich, jedoch in Vertikalrichtung - vorzugsweise jeweils gegen Federdruck -beweglich geführt gehalten sind, so daß die Dichtelemente 11 in Betriebsstellung der Vorrichtung 1 mehr oder weniger fest und damit gegen die Umgebung abdichtend auf dem Untergrund 12 aufliegen.

Um zu verhindern, daß die Dichtelemente 11 beim Transport der Vorrichtung 1 ständig am Boden scheuern, sind die Gehäuse-Segmente 6 in der Transport- bzw. Ruhestellung der Vorrichtung 1 mittels eines Stützmittels 13 in einer etwas angehobenen Ruhestellung gehalten. Dabei ist die Vertikalbeweglichkeit der Dichtelemente 11 jeweils durch einen Anschlag begrenzt, so daß die den Federdruck ausübenden Federelemente die Dichtelemente 11 nur in eine untere Grenzstellung drücken können, die während des Betriebes ausreicht, die Dichtelemente 11 gegen den Untergrund zu pressen, die jedoch in der Transport- bzw. Ruhestellung gewährleistet, daß es nicht zu einer Anlage der Dichtelemente 11 am Untergrund kommt, wenn die Gehäuse-Segmente 6 angehoben und mittels des Stützmittels 13 in dieser Stellung gehalten sind.

Bei dem Stützmittel 13 handelt es sich um eine Stützgabel, die aus einer abgesenkten Aufnahmestellung in einer angehobene Stützstellung anhebbar ist.

Wie aus der Zeichnung erkennbar ist, ist der Gehäuse-Kopfabschnitt 4 im wesentlichen kastenförmig ausgebildet, wobei seine seiner äußeren Stirnseite abgewandte, den Gehäuse-Segmenten 6 zugekehrte Rückseite sowie der Boden im wesentlichen offen ausgebildet sind, so daß die Vorrichtung 1 in Betriebsstellung (s. z.B. Fig. 2) praktisch - mit Ausnahme der einen Teil des äußersten Gehäuse-Segmentes 6.6 bildenden Rückseite - geschlossen ist.

Um auch die stirnseitige Außenseite des in Betriebsstellung an einem Ende der Vorrichtung 1 befindlichen, äußersten Gehäuse-Segmentes 6.6 beim Desinfizieren verschließen zu können, ist das äußerste Gehäuse-Segment 6.6 mittels eines Wandelementes 14 zu verschließen, bei dem es sich bei dem dargestellten Ausführungsbeispiel um eine Jalousie bzw. ein Rollo handelt, welche(s) am horizontalen Steg 15 (s. Fig. 5) des äußersten Gehäuse-Segmentes 6.6 angeordnet ist und sich aus seiner in Fig. 5 dargestellten Öffnungsstellung gemäß dem Pfeil 16 herunterziehen läßt, um die Vorrichtung 1 auch an ihrem rückwärtigen Ende zu verschließen, wie dieses in Fig. 5a erkennbar ist. Ist der noch zu beschreibende Desinfiziervorgang beendet, so läßt sich die Jalousie 14 gemäß dem Pfeil 16' (Fig. 5a) wieder in die Stellung gemäß Fig. 5 hochschieben und das Gehäuse 3 damit wiederum am rückwärtigen Ende öffnen, so daß der desinfizierte Gegenstand 2 dann wieder aus der Vorrichtung 1 herauszunehmen bzw. herauszufahren ist.

An den Innenseiten der Gehäuselängsabschnitte 4, 6 (insbesondere den Gehäuse-Segmenten 6) sind mehrere UV-Strahler 17 angeordnet, mit denen der gesamte Innenraum 18 des Gehäuses 3 mit UV-Licht zu bestrahlen ist, und zwar sind sowohl an den horizontalen Stegen der umgekehrt U-förmigen Gehäuse-Segmente 6 als auch an deren vertikalen Schenkeln UV-Strahler 17 angeordnet.

Horizontale UV-Strahler 17 im oberen und unteren Bereich des Innenraums 18 sind bspw. aus Fig. 7 zu erkennen, wobei in Fig. 7 nur jeweils ein oberer UV-Strahler 17 sowie ein unterer UV-Strahler 17 zu erkennen ist, tatsächlich aber mehrere (nämlich jeweils drei) UV-Strahler sowohl oben als auch unten vorgesehen sind, die in der Betriebsstellung (s. z.B. Fig. 2) mit gegenseitigem Abstand angeordnet sind, so daß beim Desinfizierbetrieb der gesamte Innenraum 18 des Gehäuses 3 mit UV-Licht zu durchfluten ist.

Darüber hinaus sind auch noch mehrere vertikale UV-Strahler 17 vorhanden, wie ebenfalls aus Fig. 7 erkennbar ist.

Die Innenseiten der Gehäuse-Segmente 6, des Wandelementes 14 und der dem Innenraum 18 zugekehrten Wand 19 des Gehäuse-Kopfabschnittes 4 sind jeweils mit einer vielseitig reflektierenden Metallbeflockung beschichtet, um die UV-Durchstrahlung des gesamten Innenraums 18 noch zu verbessern bzw. zu vergleichmäßigen.

Wie aus Fig. 10 weiterhin zu erkennen ist, sind die UV-Strahler 17 jeweils mit einem Schutzgitter 17' versehen.

Dabei zeigt Fig. 10a einen Seiten-UV-Strahler 17 des Gehäuse-Kopfabschnittes 4 in teilweiser Explosionsdarstellung, welche die Montage- und Demontagemöglichkeit des Schutzgitters 17' erkennen läßt und damit zugleich die Möglichkeit einer Auswechslung des Lampenkörpers des UV-Strahlers 17 zeigt.

Fig. 10b zeigt einen unteren Abschnittes des Gehäuse-Kopfabschnittes 4 sowie einen dort angeordneten, bodenseitigen UV-Strahler 17 in einer entsprechenden Darstellung wie Fig. 10a.

Das Gehäuse 3 der Vorrichtung 1 ist mit einem nach innen gerichteten Druckluftauslaß versehen und weist darüber hinaus einen nach innen gerichteten Sauganschluß auf, dem ein Filter vorgeordnet ist, wobei der Sauganschluß höher angeordnet ist als der Druckluftauslaß, so daß durch in den Innenraum 18 eingeblasene Druckluft aufgewirbelte Partikel besonders zweckmäßig aus dem Innenraum 18 entfernt werden können.

Darüber hinaus ist an der Außenseite des Gehäuses 3 ein Sauganschluß 21 vorhanden, an den ein Staubsauger 22 anzuschließen ist, der im Transport- bzw. Ruhezustand der Vorrichtung 1 raumsparend in einem Fach 23 an der vorderen Stirnwand des Gehäuse-Kopfabschnittes 4 unterzubringen ist (s. Fig. 3), welches nach Öffnen einer Schwenktür 24 zugänglich ist.

Im Innenraum 18 des Gehäuses 3 sind mehrere Sprühdüsen 26 zum Versprühen von Desinfektionsmittel vorhanden, mittels derer im Innenraum 18 ein Sprühnebel zu erzeugen ist (s. z.B. Fig. 7).

Um ein Gegenstand wie ein Bett bzw. dessen Matratze 2', dessen Breite ggf. größer ist als die lichte Breite B' des Innenraums 18 (s. z.B. Fig. 4) dennoch in die Vorrichtung 1 einbringen zu können, oder um die Einzelteile (z.B. Matratze 2' und Bettgestell 2") beim Desinfizieren so voneinander trennen zu können, daß eine wirksame Desinfizierung erfolgen kann, ist eine Kippeinrichtung 27 (s. Fig. 7) vorhanden, mittels welcher ein zu desinfizierender Gegenstand relativ zur Längsachse seitlich zu kippen und in einer Kippstellung abzustützen ist. Bei dieser Kippeinrichtung 27 kann es sich um ein bspw. aus Rohren od.dgl. bestehendes Gestell handeln, oder aber auch um eine von der Steuereinrichtung 28 aus betätigbare Hub-Kipp-Einrichtung, die bspw. aus zwei mit gegenseitigem Abstand zueinander angeordneten pneumatischen (oder hydraulischen) Kolben-Zylinder-Einheiten besteht.

Wie oben bereits ausgeführt worden ist, zeigt Fig. 1 die Vorrichtung 1 im Transport- bzw. Ruhezustand, in dem sämtliche Gehäuse-Segmente 6.1 bis 6.6 teleskopartig ineinadergefahren und von der Gabel 13 bzw. Kipphaken 13' abgestützt derart gehalten sind, daß die schürzenartigen Dichtungselemente 11 in diesem Zustand nicht auf dem Untergrund 12 aufliegen, so daß die sehr handliche Vorrichtung mühelos von einer Bedienungsperson verfahren werden kann. Um sie dabei vor Stößen zu schützen, ist an seitlichen unteren Randabschnitten des Gehäuse-Kopfabschnittes 4 sowie an dessen außenliegendem unteren Rand eine aus einem Gummiwulst bestehende Stoßwulst 29 vorgesehen.

Soll ein Bett 2 mit der Vorrichtung 1 desinfiziert werden, so wird der Bettrahmen zuvor erst einmal auf konventionelle Weise gereinigt. Sodann wird - noch im Transportzustand der Vorrichtung gemäß Fig. 1, in dem die Rückseite durch die Jalousie 14 geschlossen ist - die Vorrichtung 1 mittels eines elektrischen Kabels 30 an eine Steckdose 31 angeschlossen (s. Fig. 1a), und es wird dem Fach 23 der Staubsauger 22 entnommen, der nach dem Zusammensetzen sodann an den Sauganschluß 21 angeschlossen und in Betrieb gesetzt wird, um insbesondere den Bodenbereich des betreffenden Raumes von Staub zu befreien.

Danach wird die Jalousie 14 in Richtung des Pfeiles 16' (Fig. 5a) geöffnet und die betreffende Bedienungsperson erfaßt einen an der Außenseite des äußersten Gehäuse-Segmentes 6.6 angeordneten Handgriff 32, um die Vorrichtung 1 betriebsfertig zu machen, indem sie die Gehäuse-Segmente 6.6, 6.5, 6.4 etc. nacheinander in Richtung der Pfeile 33 herauszieht. Zu diesem Zwecke sind i.ü. noch weitere Handgriffe 32 an den vertikalen Schenkeln des äußersten Gehäuse-Segmentes 6.6 vorgesehen.

Nach dem Ausziehen befindet sich die Vorrichtung 1 sodann im Betriebszustand gemäß Fig. 2 bzw. Fig. 5, so daß der zu reinigende Gegenstand, bei dem es sich bei dem dargestellten Ausführungsbeispiel um ein Bett 2 handelt, in den Innenraum 18 des Gehäuses 3 hineingefahren werden kann.

Bevor die Jalousie 14 in Richtung des Pfeiles 16 (s. Fig. 5) geschlossen und demgemäß der Zustand gemäß Fig. 5a erreicht wird, wird die Matratze 2' noch mittels der Kippeinrichtung 27 angehoben, wie dieses in Fig. 7 mit strichpunktierten Linien angedeutet ist.

Nach dem Schließen des Gehäuses 3 setzt sodann der erste Reinigungsvorgang ein, indem durch den nach innen gerichteten Sauganschluß bzw. die nach innen gerichteten Sauganschlüsse der Innenraum 18 sowie das Bett 2 staubfrei gesaugt werden. Um diesen Arbeitsgang zu unterstützen bzw. zu intensivieren, kann dabei gleichzeitig durch die unterhalb der Sauganschlüsse liegenden, nach innen gerichteten Druckluftanschlüsse Luft in den Innenraum 18 eingeführt werden, welche die im Innenraum vorhandenen Staubpartikel aufwirbelt, so daß sie sodann wirksam abgesaugt werden. Dieser Vorgang dauert ca. 5 min.

Nach dieser Saug-Vorreinigung werden sodann die UV-Strahler 17 eingeschaltet, wobei die horizontal angeordneten, oberen UV-Strahler 17, von denen wenigstens zwei Strahler mit einer Leistung von jeweils 65 W vorhanden sind, sowie die an den Seitenwänden vertikal angeordneten UV-Strahler 17, von denen vier Strahler 17 mit einer Leistung von jeweils 30 W vorhanden sind, den Innenraum 18 des Gehäuses vollständig intensiv mit UV-Strahlen durchfluten, so daß das UV-Licht eine wirksame Desinfizierung vornehmen kann. Dieses wird nicht zuletzt deshalb dadurch erreicht bzw. unterstützt, daß die Innenwände des Gehäuses 3 mit einer Metallbeflockung beschichtet sind, so daß sich eine intensive Reflexion der auf die Seitenwände auftreffenden UV-Strahlen nach allen möglichen Richtungen ergibt.

Aus Sicherheitsgründen sind die UV-Strahler 17 nur dann einzuschalten bzw. eingeschaltet zu halten, wenn das Wandelement 14 sich in Schließstellung befindet. Ist die Jalousie 14 versehentlich offengelassen worden oder wird sie während der UV-Bestrahlung geöffnet, so werden die UV-Strahler 17 gar nicht erst eingeschaltet bzw. abgeschaltet.

Für die UV-Bestrahlung reicht ein Zeitraum von ca. 3 min völlig aus.

Bei der nachfolgenden chemischen Desinfizierung wird ein geeignetes flüssiges Desinfektionsmittel (z.B. Ilgasa DP 300 in Verdünnung mit Äthanol) mittels der Sprühdüse (n) 26 in den Innenraum 18 eingesprüht, wobei aufgrund der Ausgestaltung der Sprühdüse (n) 26 (s. Fig. 9) sowie der Verdünnung mit Äthanol eine regelrechte Vernebelung erfolgt, die einerseits zu einer sehr wirksamen Desinfizierung und andererseits nicht zu einer unnötigen Befeuchtung des Bettes 2 (insbesondere dessen Matratze 2') führt.

Nachdem für etwa 2 min Desinfiziermittel eingesprüht worden ist, folgt eine Ruhezeit von ca. 5 min, in der sich der Sprühnebel mit Sicherheit vollständig gesetzt hat, so daß danach die Jalousie 14 wieder geöffnet und das Bett in optimal desinfiziertem Zustand aus der Vorrichtung 1 herausgefahren werden kann.

Insgesamt dauert der Desinfiziervorgang mithin höchstens 15 min, wobei das Bett 2 anschließend unverzüglich bezogen und neu belegt werden kann.

Mit der erfindungsgemäßen Desinfiziervorrichtung liegt mithin nunmehr ein sowohl in technischer als auch in wirtschaftlicher Hinsicht in höchstem Maße befriedigendes, mobiles Mittel vor, mit dem in Krankenhäusern od.dgl, insbesondere Betten, aber auch andere Gegenstände, schnell und optimal zu desinfizieren sind.

Es bleibt noch nachzutragen, daß die Vorrichtung 1 nach erfolgter Desinfizierung sodann wieder in umgekehrter Reihenfolge in den Transport- bzw. Ruhezustand gemäß Fig. 1 überführt wird, wenn in dem betreffenden Raum nicht noch andere Gegenstände desinfiziert werden sollen bzw. wenn sämtliche zu desinfizierenden Gegenstände eines Raumes desinfiziert worden sind, so daß die Vorrichtung 1 danach dann sogleich zu einer anderen Stelle verfahren werden kann, an welcher Desinfizierarbeiten durchzuführen sind.

Es sei noch nachgetragen, daß dem in der Wand 19 des Gehäuse-Kopfabschnittes 4 angeordneten Sauganschluß ein mehrteiliger Filter vorgeordnet ist, der aus einer mit Durchgangsbohrungen 35 versehenen Deckplatte 36 (s. Fig. 8), einem der Deckplatte 36 in Strömungsrichtung nachgeordneten Vorfilter 37 und einen dem Vorfilter 37 in Saugrichtung nachgeordneten Nachfilter (Hepa-Filter 38) besteht, wobei die Befestigung der insgesamt den Filter 34 bildenden Teile 36 bis 38 mittels vier Schrauben 39 an der Wand 39 erfolgt.

Unter Bezugnahme auf Fig. 11 sei noch darauf verwiesen, daß mit verschiedenen Testen Bakterien gezüchtet und auf einer Testmatratze 2' verteilt worden sind, die sodann in die Vorrichtung 1 (ohne Kippung) eingebracht und desinfiziert worden ist, und zwar Escherechia coli IFO 3301 (E. coli), Staphylococcus aureus IFO 13276 (S. aureus) und Bacillus subtilis ATCC 6633 (B. subtilis), wobei die entsprechenden Microorganismen-Subspensionen von E. coli und S. aureus nach Beimpfung eines Nährbodens eine Nacht lang bei 37° C entwickelt wurden und die Körper der Microorganismen in einer sterilisierten physiologischen Salzlösung dann beflutet wurden. Bgzl, B. subtilis wurde ebenfalls ein Nährboden beimpft und unter 37° C gehalten, wobei die Sporen der Microorganismen ebenfalls entsprechend behandelt und sodann erwärmt wurden.

Die Microorganismen-Suspensionen wurden sodann auf Testmedien gegeben, so daß sich die Kolonien dort wenigstens verdoppeln konnten und sodann auf dem zu prüfenden Bett 2 bzw. dessen Matratze 2' durch Aufstreichen verteilt.

Nach Durchführung einer Desinfizierung mit der erfindungsgemäßen Vorrichtung konnten keine entsprechenden Microorganismen mehr festgestellt werden; die Desinfizierung war mithin optimal.

Die in der vorstehenden Beschreibung, in der Zeichnung sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## BEZUGSZEICHENLISTE

### (LIST OF REFERENCE NUMERALS)

1 Desinfiziervorrichtung
2 Betten
2' Matratze (von 2)
2" Bettgestell (von 2)
3 Gehäuse (von 1)
4 Gehäuse-Kopfabschnitt
6 Gehäuse-Segmente
7 Pfeil (Längsrichtung von 1)
8 Laufrollen (von 4)
9 Laufröllchen (von 6)
11 Dichtelemente (von 6)
12 Untergrund
13 Gabel
13' Kipphaken
14 Wandelement (Jalousie bzw. Rollo)
15 Steg (von 6.6)
16, 16' Pfeile
17 UV-Strahler
17' Schutzgitter (für 17)
18 Innenraum (von 3)
19 Wand (von 4)
21 Sauganschluß
22 Staubsauger
23 Fach
24 Schwenktür
26 Sprühdüse (n)
27 Kippeinrichtung
28 Steuereinrichtung
29 Stoßschutz
30 Kabel

31 Steckdose
32 Handgriff
33 Pfeile
34 Filter
35 Durchgangsbohrungen (von 36)
36 Deckplatte
37 Vorfilter
38 Nachfilter
39 Schrauben

## Ansprüche

1. Vorrichtung zum Desinfizieren von insbesondere in Krankenhäusern od.dgl. befindlichen Gegenständen wie Rollstühlen, Strecktischen, Rehabilitationsgeräten, Instrumentenwagen etc., insbesondere Betten, mit einem verschließbaren Gehäuse, in dem wenigstens eine Desinfiziereinrichtung angeordnet ist, dadurch gekennzeichnet, daß das auf Rollen (8, 9) od.dgl. verfahrbare Gehäuse (3) aus wenigstens zwei Längsabschnitten (4, 6) besteht, die aus ihrer Betriebsstellung (z.B. Fig. 2) in Längsrichtung (7) der Vorrichtung (1) unter Verkürzung der Betriebslänge (L) der Vorrichtung (1) relativ zueinander beweglich sind.

2. Vorrichtung nach Anspruch 1, dadurch gekennezeichnet, daß die Gehäuse-Längsabschnitte (4, 6) teleskopierbar ausgebildet sind.

3. Vorrichtung nach Anspruch 1, dadurch gekennzeichnet, daß wenigstens ein Gehäuse-Längsabschnitt (6) als Faltenbalg od.dgl. ausgebildet ist.

4. Vorrichtung nach einem oder mehreren der vorhergehenden Ansprüche, dadurch gekennzeichnet, daß das Gehäuse (3) aus mehr als zwei Gehäuse-Abschnitten (4, 6.1, 6.2, ..., 6.6) besteht.

5. Vorrichtung nach Anspruch 4, dadurch gekennzeichnet, daß das Gehäuse (3) einen Gehäuse-Kopfabschnitt (4) sowie mehrere (z.B. sechs) Gehäuse-Segmente (6) aufweist.

6. Vorrichtung nach Anspruch 5, dadurch gekennzeichnet, daß der Gehäuse-Kopfabschnitt (4) sowie die Gehäuse-Segmente (6) jeweils auf Laufrollen (8; 9) abgestützt verfahrbar sind.

7. Vorrichtung nach Anspruch 6, dadurch gekennzeichnet, daß die Gehäuse-Segmente (6) in der Transport- bzw. Ruhestellung der Vorrichtung (1) mittels eines Stützmittels (13) in einer angehobenen Ruhestellung zu halten sind.

8. Vorrichtung nach Anspruch 7, dadurch gekennzeichnet, daß das Stützmittel (13) in der Art einer Stützgabel ausgebildet ist.

9. Vorrichtung nach Anspruch 8, dadurch gekennzeichnet, daß die Stützgabel (13) od.dgl. aus einer abgesenkten Aufnahme stellung in eine Haltestellung für die Gehäuse-Segmente (6) anhebbar ist.

10. Vorrichtung nach einem oder mehreren der vorhergehenden Ansprüche, dadurch gekennzeichnet, daß die Gehäuse-Segmente (6) umgekehrt U-förmig ausgebildet sind.

11. Vorrichtung nach einem oder mehreren der vorhergehenden Ansprüche, insbesondere nach Anspruch 10, dadurch gekennzeichnet, daß jeweils an den unten befindlichen, freien Randabschnitten der Gehäuse-Segmente (6) schürzenförmige Dichtelemente (11) angeordnet sind, die sich in der Betriebsstellung der Vorrichtung (1) im wesentlichen bis zum Untergrund (12) erstrecken.

12. Vorrichtung nach Anspruch 11, dadurch gekennzeichnet, daß die Dichtelemente (11) in Längsrichtung (7) der Vorrichtung (1) unbeweglich und in Vertikalrichtung - vorzugsweise gegen Federdruck - beweglich geführt gehalten sind.

13. Vorrichtung nach einem oder mehreren der vorhergehenden Ansprüche, dadurch gekennzeichnet, daß die stirnseitige Außenseite des in Betriebsstellung an einem Ende der Vorrichtung (1) befindlichen, äußeren Gehäuse-Segmentes (6. 6) mittels wenigstens eines Wandelementes (14) zu verschließen ist.

14. Vorrichtung nach Anspruch 13, dadurch gekennzeichnet, daß das Bandelement (14) des äußersten Gehäuse-Segmentes (6. 6) ein am horizontalen Steg des Gehäuse-Segmentes (6. 6) angeordnetes Rollo, eine Jalousie od.dgl. ist.

15. Vorrichtung nach einem oder mehreren der vorhergehenden Ansprüche, dadurch gekennzeichnet, daß an den Innenseiten der Gehäuse-Längsabschnitte (4, 6) mehrere UV-Strahler (17) angeordnet sind, mit denen im wesentlichen der gesamte Innenraum (18) des Gehäuses (3) mit UV-Licht zu bestrahlen ist.

16. Vorrichtung nach Anspruch 15, dadurch gekennzeichnet, daß an den horizontalen Stegen und/oder den vertikalen Schenkeln der umgekehrt U-förmigen Gehäusesegmente (6) und/oder des Gehäuse-Kopfteils (4) UV-Strahler (17) angeordnet sind.

17. Vorrichtung nach einem oder mehreren der vorhergehenden Ansprüche, dadurch gekennzeichnet, daß die Innenseiten der Gehäuse-Segmente (6) und/oder die Innenseite des Wandelementes (14) und/oder die dem Innenraum (18) zugekehrte Seite der Wand (19) des Gehäuse-Kopfteils (4) mit einer vielseitig reflektierenden Metallbeflockung od.dgl. beschichtet sind.

18. Vorrichtung nach einem oder mehreren der vorhergehenden Ansprüche, dadurch gekennzeichnet, daß das Gehäuse (3) mit wenigstens einem nach innen gerichteten Druckluftauslaß versehen ist.

19. Vorrichtung nach einem oder mehreren der vorhergehenden Ansprüche, dadurch gekennzeichnet, daß das Gehäuse (3) mit wenigstens einem nach innen gerichteten Suganschluß versehen ist.

20. Vorrichtung nach Anspruch 18 und 19, dadurch gekennzeichnet, daß der Sauganschluß höher angeordnet ist als der Druckluftauslaß.

21. Vorrichtung nach einem oder mehreren der Ansprüche 18 bis 20, dadurch gekennzeichnet, daß dem Sauganschluß ein Filter (34) vorgeordnet ist.

22. Vorrichtung nach einem oder mehreren der vorhergehenden Ansprüche, dadurch gekennzeichnet, daß an der Außenseite des Gehäuses (3) wenigstens ein Sauganschluß (21) vorhanden ist, an den ein Staubsauger (22) anzuschließen bzw. angeschlossen ist.

23. Vorrichtung nach einem oder mehreren der vorhergehenden Ansprüche, dadurch gekennzeichnet, daß im Innenraum (18) des Gehäuses (3) mehrere Sprühdüsen (26) zum Versprühen eines flüssigen Desinfizierungsmittels angeordnet sind.

24. Vorrichtung nach einem oder mehreren der vorhergehenden Ansprüche, gekennzeichnet durch wenigstens eine Kippeinrichtung (27), mittels welcher ein zu desinfizierender Gegenstand (2') relativ zur Längsachse seitlich zu kippen und in einer Kippstellung anbzustützen ist (Fig. 7).

25. Vorrichtung nach einem oder mehreren der vorhergehenden Ansprüche, gekennzeichnet durch eine ggf. einen Rechner aufweisende Steuereinrichtung (28), mittels welcher der Ablauf wenigstens eines Desinfizierungsprogramms automatisch zu steuern ist.

26. Vorrichtung nach einem oder mehreren der Ansprüche 5 bis 25, dadurch gekennzeichnet, daß der Gehäuse-Kopfabschnitt (4) im wesentlichen kastenförmig ausgebildet ist, wobei seine seiner äußeren Stirnseite abgewandte, den Gehäuse-Segmenten (6) zugekehrte Rückseite sowie der Boden im wesentlichen offen ausgebildet sind, und daß im Bereich der geschlossenen Gehäuseseiten Fächer (z.B. 23) u.dgl. zur Aufnahme von Geräten (z.B. 22) vorhanden sind.

27. Vorrichtung nach einem oder mehreren der Ansprüche 15 bis 26, dadurch gekennzeichnet, daß die UV-Strahler (17) nur dann einzuschalten sind, wenn das Wandelement (14) sich in seiner Schließstellung befindet.

EP 0 366 827 A1

**FIG.1**

**FIG.1a**

**FIG.2**

**FIG.3**

**FIG.4**

**FIG.5**

**FIG.6**

**FIG.5a**

**FIG.7**

**FIG.11**

**FIG.8**

**FIG.9**

**FIG.10**

a)

b)

c)

d)

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-2 913 324  (WEXIÖDISK) --- | | A 61 L   2/00 A 61 L   2/10 |
| A | FR-A-2 608 431  (J.L. SALLAZ) ----- | | |

RECHERCHIERTE
SACHGEBIETE (Int. Cl.5)

A 61 L

Der vorliegende Recherchenbericht wurde für alle Patentansprüche erstellt

| Recherchenort | Abschlußdatum der Recherche | Prüfer |
|---|---|---|
| DEN HAAG | 12-06-1989 | COUSINS-VAN STEEN G.I.L. |

EPO FORM 1503 03.82 (P0403)